Europäisches Patentamt

**European Patent Office**

Office européen des brevets

(19)

(11) Publication number: **0 116 420**
**A2**

(12) # EUROPEAN PATENT APPLICATION

(21) Application number: **84300432.6**

(22) Date of filing: **25.01.84**

(51) Int. Cl.³: **A 61 B 5/16**

(30) Priority: **26.01.83 AU 7771/83**

(43) Date of publication of application:
**22.08.84 Bulletin 84/34**

(84) Designated Contracting States:
**AT BE CH DE FR GB IT LI LU NL SE**

(71) Applicant: **Lesur, Adrien Louis**
**41 Wentworth Street**
**Tempe New South Wales 2044(AU)**

(72) Inventor: **Lesur, Adrien Louis**
**41 Wentworth Street**
**Tempe New South Wales 2044(AU)**

(74) Representative: **Jukes, Herbert Lewis et al,**
**Swann, Elt & Company 31 Beaumont Street**
**Oxford OX1 2NP(GB)**

(54) **Digital dexterity analyser.**

(57) The present invention discloses a digital dexterity ana-lyser which enables the speed of finger movement to be measured. Such a device is of benefit to arthritics, persons recovering from surgery to the hands and/or fingers, musi-cians, sportsmen or the like.

A manually held probe is moved between two electrodes (14-19) and a measure of the number of movements made within a predetermined time determined by a timer (32) is recorded in a counter (33) and displayed in a display (3).

The device is also useful as an educational aid in the teaching of calligraphy by means of a guide attachable to the device.

FIG.3

EP 0 116 420 A2

## "DIGITAL DEXTERITY ANALYSER"

The present invention relates to a measuring device which measures the speed of finger movement and which will hereinafter be referred to as a dexterity analyser. Such analysers are used to monitor the progress and development of the finger and digital muscles and their operation.

Persons suffering from ailments such as arthritis, recovering from surgery to the hands and fingers, and the like, can lose control of the motor functions of their fingers. In particular, this can lead to the inability to control writing implements. A dexterity analyser allows occupational health staff to keep a statistical record of the progress and development of such persons. Such apparatus also provides a tool or exercise device by which persons such as pianists, sportsmen, and the like can improve and strengthen the motor functions of the fingers and wrists.

A dexterity analyser can also be utilised in the role of a toy, which has the added benefit of helping a player develop his or her writing capabilities. This is especially valuable for children.

A dexterity analyser also has a substantial potential usage as a educational device in teaching persons how to write characters, whether in their own or another language, recognise letters of other alphabets, count in binary fashion, and the like.

- 2 -

It is the object of the present invention to provide a dexterity analyser.

According to one aspect of the present invention there is disclosed a manual dexterity analyser comprising first and second shaped electrodes located adjacent each other and electrically insulated from each other, a conductive manually held probe, and an electric circuit; wherein said electric circuit comprises a timer means and a counter means both connected to said electrodes, said probe is connected to a predetermined voltage potential used in said electric circuit, initial movement of said probe from one of said electrodes to the other activates said timer for a predetermined period and continuation of said movement increments the contents of said counter to record a measure of the number of said movements during said predetermined period.

An embodiment of the present invention will now be described with reference to the drawings in which:-

Fig. 1 is a perspective view of the dexterity analyser of the preferred embodiment;

Fig. 2 is a perspective view of an attachment for use with the analyser of Fig. 1;

Fig. 3 is a schematic block diagram of the circuit of the analyser of Fig. 1; and

Figs. 4 and 5 are circuit diagrams of the actual circuit of the analyser of Fig. 1.

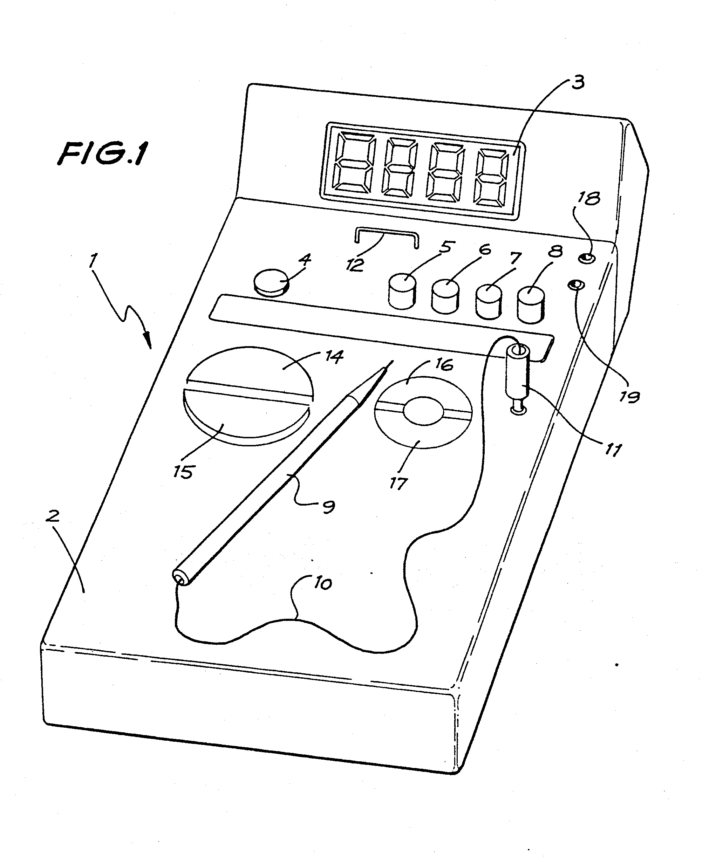

Turning now to Fig. 1, the dexterity analyser 1 comprises a housing 2 having a digital display 3 and on/off button 4 which controls an electric circuit powered by a battery (both not illustrated) located within the housing 2.

Also located on the housing 2 are four timer buttons 5 to 8 respectively and a conductive probe 9 which is connected by means of a flexible lead 10 and a banana plug 11 to the electric circuit inside the housing 2. A reset contact 12 is provided in the form of an inverted U-shaped wire which forms one contact of the internal electric circuit.

The housing 2 is provided with three pairs of electrodes 14 to 19 which are electrically connected in parallel. The first and second electrodes 14 and 15 each comprise a semi-circular disc of metal, the discs being mounted so as to be co-planar and facing each other so as to nearly form a circle. A small insulating gap is provided between the electrodes 14 and 15.

The second pair of electrodes comprises a third and fourth electrode 16,17 which are each semi-annular in shape and are mounted so as to be co-planar and facing each other so as to substantially form an annulus. The electrodes 16 and 17 are insulated from each other and define a substantially circular path separated by two insulative gaps. The third pair of electrodes comprises fifth and sixth electrodes 18,19 which permit an external connection of a desired pair of electrodes.

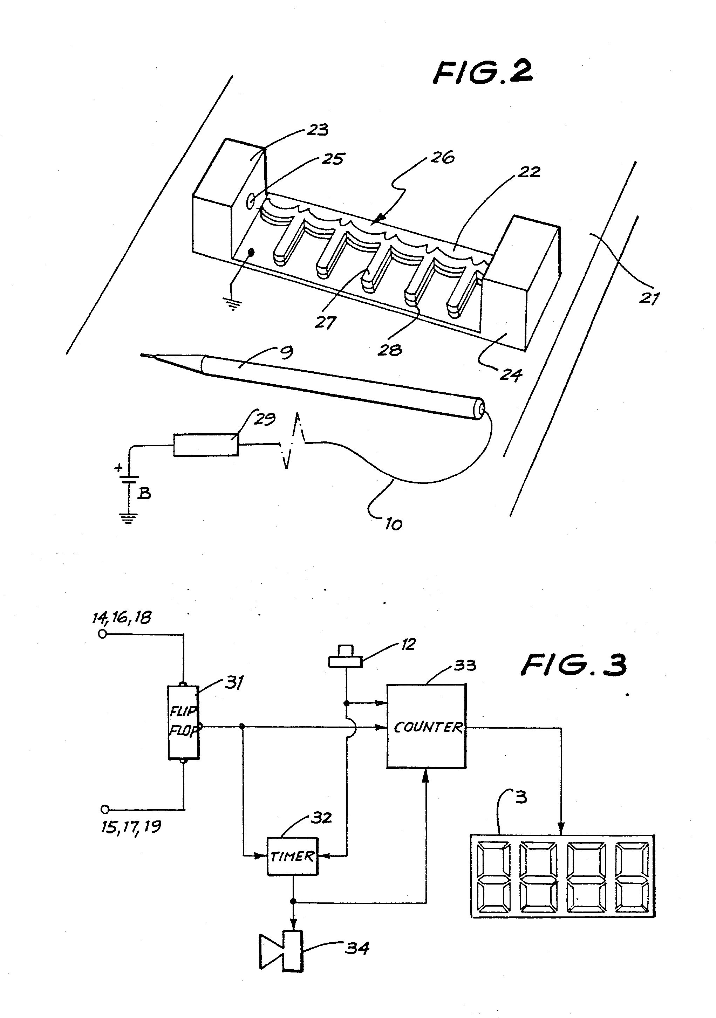

An attachment for use with the analyser 1 of Fig. 1 is illustrated in Fig. 2. The attachment comprises a board 21 made from insulative material and upon which is mounted a metal earthing strip 22 which extends between two housings 22,23 which each contain a beam coupling device 25 (only one being illustrated) which provides for a beam of light or other electromagnetic radiation, such as infra-red, to extend between the housings 23,24. Also extending between the housings 23,24 is a shaped calligraphy guide 26 which is cut from two laminated sheets so as to form an upper conductive layer 27 of metal and a lower insulative layer 28 formed from a suitable plastics material. The calligraphy guide 26 is shaped to form a series of lower case r's joined together. Means are provided (but not illustrated) to connect the upper layer 27 to the housing 2 via, say, the electrode 18.

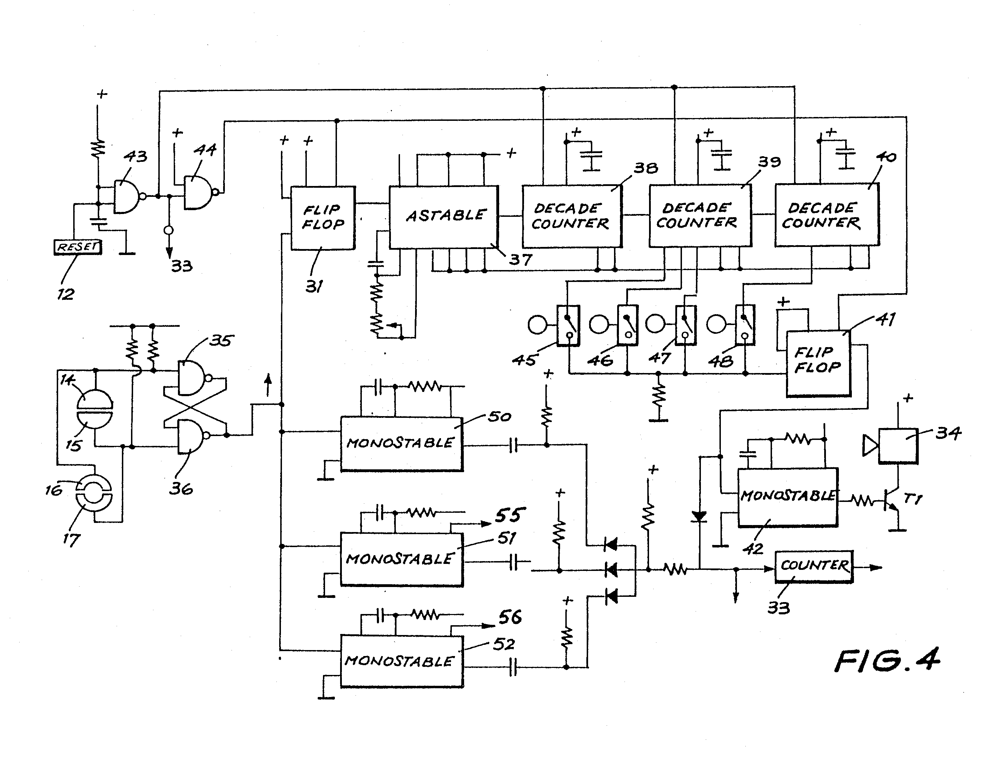

Turning now to Fig. 3, the schematic electric circuit housed within the dexterity analyser 1 is illustrated and comprises a flip flop 31, a timer 32, a counter 33, a buzzer 34, the digital display 3 and a schematically illustrated reset contact 12.

In use, the manual dexterity analyser 1 is first activated by depressing the on/off button 4, the probe 9 is held in the fingers in the same manner as a pencil or pen is held and a series of circles or ellipses are traced out by passing the tip of the probe to and fro across the surfaces of the electrodes 14,15 (for example). The motion of the fingers in drawing lines or loops across the electrodes 14,15 is very substantially dependent upon the conditioning of the muscles of the fingers and also to some extent the wrists. It is estimated that approximately 300 exchanges per minute between the electrodes 14,15 are required if legible handwriting is to be achieved.

By depressing one of the timer buttons 5 to 8 a timing period of twenty seconds, thirty seconds, sixty seconds or five minutes, respectively, can be preselected. The motion of the tip of the probe 9 from, say, electrode 14 to electrode 15 and back to electrode 14 activates the flip flop 31 of Fig. 3 and sends a corresponding count signal to the counter 33. The initial count signal produced by the flip flop 31 also activates the timer 32. Continued motion of the probe 9 increases the score accumulating in the counter 33 until expiration of the predetermined time period. At that time the buzzer 34 is sounded and the counter 33 is prevented from any further increase in count. Preferably the counter is arranged so that during the twenty and thirty second preselected periods the count shown is not the actual count but the count rate per minute. Clearly, for the sixty second preselected interval the count rate per minute and the actual count are identical. For the five minute selected period the actual count is displayed.

At the end of the preselected period after the score has been noted, both the counter and the timer are re-set by use of the reset contact 12 and the dexterity analyser 1 is then ready for another operation.

If the probe 9 is used on the electrodes 16,17 then a harder test of manual dexterity results. This is because the tip of the probe 9 must follow a path located within the annulus defined by the electrodes 16,17 in order to register a score. In consequence, persons using the electrodes 16,17 generally score a lower count than when the electrodes 14,15 are used.

When the attachment of Fig. 2 is utilised together with the housing 1 of Fig. 1, the electrical circuit connected to the probe 9 can be represented by a battery B connected in series with a resistor 29. The purpose of the test utilising the calligraphy guide 26 is for the operator to have the tip of the probe 9 trace out a series of connected lower case r's by tracing the outline provided by the shaped upper layer 27. Should the tip of the probe 9 fall from the upper layer 27 and contact the earthing strip 22, then the alarm 29 is sounded so as to indicate an error.

It will be appreciated that the abovementioned arrangement finds particular application in the correction of handwriting faults since it provides a means with immediate correction of errors whereby difficult letters can be formed repeatedly in the correct manner. It will also be appreciated that the apparatus of Fig. 2 can be utilised to teach the writing of, for example, Chinese, Japanese and Arabic characters with a suitably shaped calligraphy guide 26.

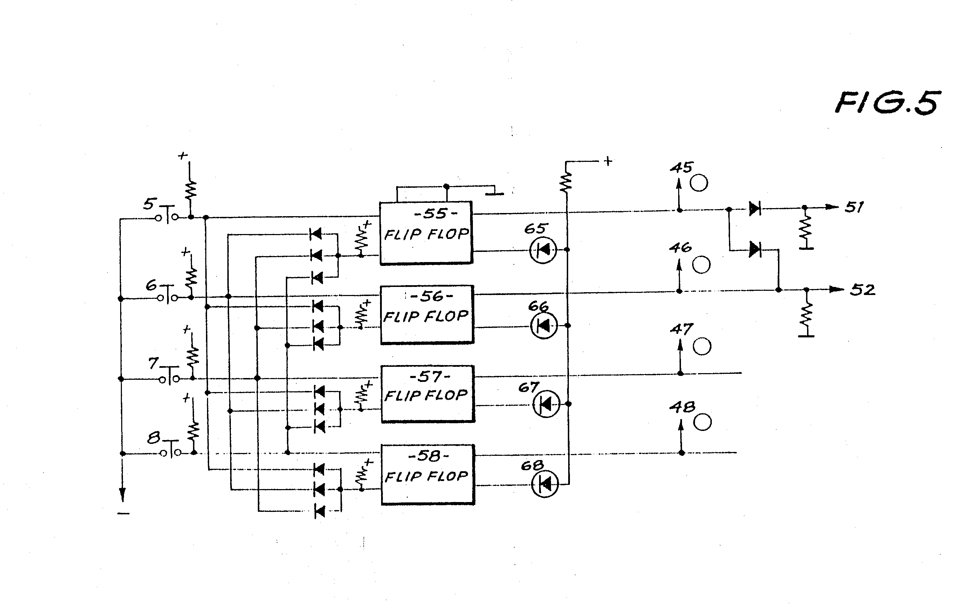

An actual circuit realisation of the schematic circuit of Fig. 3 is illustrated in Figs. 4 and 5. The electrodes 14,15 and 16,17 are connected in parallel between the inputs of two cross coupled NAND gates 35,36 which in this configuration operate to reduce contact bounce caused by inadvertent momentary lifting of the tip of the probe 19 from the surface of, say, electrode 14. The series of pulses derived from the NAND gates 35,36 are passed to the flip flop 31 so that the first such pulses triggers an astable multivibrator 37 which is arranged to oscillate at a reference frequency or reference rate.

The output of the astable 37 is passed to three series connected decade counters 38, 39 and 40 having counting outputs which are directly connected to four bilateral switches 45 to 48 which correspond with the timer buttons 5 to 8 respectively. The output from the bilateral switches 45 to 48 is connected to a further flip flop 41 the output of which is then passed to both the counter 33 and a monostable 42 which in turn is used to activate the buzzer 34 by switching a transistor T1 ON. The output of the NAND gates 35,36 is also passed to three parallel connected monostables 50, 51 and 52.

The monostables 51 and 52 have CLEAR lines which are connected to the outputs of flip flops 55 to 58 (Fig. 5) which correspond to the timer buttons 5 to 8 respectively and are respectively connected thereto. Thus after timer button 5 has been pressed both monostables 51 and 52 are maintained CLEARED for the predetermined period. Similarly pressing button 6 maintains monostable 52 CLEARED. Also connected to the flip flops 55 to 58 is a respective one of four LED's 65 to 68 respectively, which are mounted within the timer buttons 5 to 8.

When one of the timer buttons 5 to 8 is depressed, the corresponding one of the flip flops 55 to 58 is activated thereby activating the corresponding LED 65 to 68 and also activating the corresponding one of the bilateral switches 45 to 48.

When operation of the probe 9 on the electrodes 14,15 (for example) commences, an initial pulse from the NAND gates 35,36 activates the flip flop 31 and thereby enables the astable 37 the output of which is received by the decade counters 38, 39 and 40. After a period of time determined by the pulse repetition rate of the astable 37 and the predetermined count value of the counters 38, 39 and 40 connected to the selected one of the bilateral switches 45-48 the flip flop 41 is activated thereby simultaneously activating the monostable 22 so as to sound the buzzer 34 and also disabling the counter 33. This signifies the end of the preselected time period.

During the preselected time period, pulses from the NAND gates 35,36 have been passed to the monostables 50, 51 or 52 which, if not CLEARED, in turn have passed a corresponding pulse train for each output pulse of the NAND gates 35,36, the pulse train being received by the counter 33. The monostable 50 is so arranged that when the twenty second timer button 5 is depressed, the pulses from the NAND gates 35,36 are effectively trebled by providing three pulses from the monostable 50 for each pulse produced by the NAND gates 35,36. In this way the count rate is effectively trebled to give a rate per minute displayed by the digital display 3. Similarly, in the event that the timer button 6 is depressed, the monostable 51 is arranged to give a pulse train having two pulses for each of the pulses produced by the NAND gates 35,36. Since the timer buttons 7 and 8 are required to record a direct count, the monostable 52 provides a single pulse in its pulse train for each pulse received from the NAND gates 35,36.

It will be appreciated that because of the diode coupling between the monostables 50, 51 and 52 the pulses from the longer or longest pulse train will be passed to the counter 33. In addition, the diode coupling between the flip flop 41 and the counter 33, ensures that when the flip flop 41 is activated this effectively uncouples the monostables 50, 51 and 52 from the counter 33 and therefore no further counts are recorded.

A further pair of NAND gates 43,44 are provided connected to the reset contact 12 to enable the decade counters 38, 39 and 40 and flip flop 41 to be reset at the end of a counting period. Similarly, the counter 33 is also reset. The reasliation of the circuit components by actual integrated circuits is set out in Table 1.

TABLE 1

| ITEM NO | NAME | INTEGRATED CIRCUIT | | MANUFACTURER |
|---|---|---|---|---|
| 3 | Digital Display | 1xMM74C926 | Four Digit Counter with Segment Output Driver | Motorola |
| 33 | Counter | | | |
| 35,36,43,44 | NAND gates | 1x74LS00 | Law Schottky Quad Two input NAND gate | National |
| 37 | Astable | 1x4047 | Astable Multivibrator | " |
| 31,41,56-58 | Flip Flops | 6x74C74 | Dual D Flip Flop | " |
| 38,39,40 | Decade Counters | 3x4017 | Decade Counter | " |
| 45,46,47,48 | Bilateral Switches | 1x4066 | Quad Bilateral Switch | " |
| 41,50,51,52 | Monostables | 4x74C221 | Dual Multivibrator | " |

The foregoing describes only one embodiment of the present invention and modifications, obvious to those skilled in the art, can be made thereto without departing from the scope of the present invention.

For example, if the counter 33 and digital display 3 are arranged so as to provide an output in binary characters, then the apparatus of the present invention can be used to teach students the nature of the binary number system. An equivalent modification which enables the display to cycle through the various letters of the English alphabet, Russian or Greek alphabets can also enable the apparatus to be used to introduce students to such alphabets. Alternatively, if a plurality of electrodes are provided in the form of a sequence or a grid, then the display can be adjusted to record the letter of the alphabet corresponding to that one of a sequence of, say, 26 electrodes, touched by the probe. Similarly, the display could indicate the squares available for movement of a particular nominated chess piece located at a particular one of the squares of the array of electrodes. It will also be appreciated that the arrangement shown in Fig. 2 for correctly indicating the movement of the tip of the probe 9 along a path corresponding to a sequence of lower case r's can also be adapted to indicate correct movement along a musical score, or a mathematical or chemical formula.

Claims

1. A manual dexterity analyser comprising first and second shaped electrodes (14,15) located adjacent each other and electrically insulated from each other, a conductive manually held probe (9), and an electric circuit; wherein said electric circuit comprises a timer means (32) and a counter means (33) both connected to said electrodes, said probe (9) is connected to a predetermined voltage potential used in said electric circuit, initial movement of said probe from one of said electrodes to the other activates said timer means (32) for a predetermined period and continuation of said movement increments the contents of said counter means (33) to record a measure of the number of movements during said predetermined period.

2. An analyser as claimed in claim 1, wherein said counter means records the total number of movements during said predetermined period.

3. An analyser as claimed in claim 1, wherein said counter means records the rate at which said movements are made during said predetermined interval.

4. An analyser as claimed in any one of claims 1 to 3, wherein said first and second electrodes each comprise a semi-circular disc (14,15), said electrodes being co-planar and facing each other to form a circle.

5. An analyser as claimed in claim 4, having third and fourth mutually insulated electrodes (16,17) respectively connected in parallel with said first and second electrodes, said third and fourth electrodes each comprising a planar, semi-annulus (16,17), said third and fourth electrodes being co-planar and facing each other to form an annulus.

6. An analyser as claimed in claim 5 having fifth and sixth mutually insulated electrodes (18,19) respectively connected in parallel with said first and second electrodes, said fifth electrode comprising a base plate (22) and said sixth electrode comprising shaped indicia (26) located above, and insulated from, said base plate.

7. A modification to the analyser as claimed in claim 6, wherein said analyser has two electrodes comprising said third and fourth electrodes or said fifth and sixth electrodes; or has four electrodes comprising said third to sixth electrodes respectively.

8. An analyser as claimed in any one of claims 1 to 7, wherein said counter means includes a display (3) which records said measure as a decimal number.

FIG.1

0116420

2/4

FIG.2

FIG.3

FIG.4

FIG.5